(19) Europäisches Patentamt
European Patent Office
Office européen des brevets

(11) EP 0 770 051 B1

(12) FASCICULE DE BREVET EUROPEEN

(45) Date de publication et mention de la délivrance du brevet:
**23.08.2000 Bulletin 2000/34**

(21) Numéro de dépôt: **96916193.4**

(22) Date de dépôt: **10.05.1996**

(51) Int Cl.[7]: **C07C 45/46**, C07C 49/84, B01J 29/40

(86) Numéro de dépôt international:
**PCT/FR96/00717**

(87) Numéro de publication internationale:
**WO 96/35656 (14.11.1996 Gazette 1996/50)**

# (54) PROCEDE D'ACYLATION D'ETHERS AROMATIQUES

VERFAHREN ZUR HERSTELLUNG VON AROMATISCHEN ETHERN

AROMATIC ETHER ACYLATION PROCESS

(84) Etats contractants désignés:
**BE CH DE ES FR GB IT LI NL**

(30) Priorité: **12.05.1995 FR 9505683**

(43) Date de publication de la demande:
**02.05.1997 Bulletin 1997/18**

(73) Titulaires:
- **RHODIA CHIMIE**
  **92408 Courbevoie Cédex (FR)**
- **INSTITUT FRANCAIS DU PETROLE**
  **92506 Rueil Malmaison Cédex (FR)**

(72) Inventeurs:
- **SPAGNOL, Michel**
  **F-69003 Lyon (FR)**
- **GILBERT, Laurent**
  **F-75015 Paris (FR)**
- **BENAZZI, Eric**
  **F-78360 Montesson (FR)**
- **MARCILLY, Christian**
  **F-78800 Houilles (FR)**

(74) Mandataire: **Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**EP-A- 0 334 096**
**EP-A- 0 455 332**
**EP-A- 0 459 495**
**EP-A- 0 488 867**
**FR-A- 2 667 063**
**US-A- 3 308 069**
**US-A- 5 200 168**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 770 051 B1

## Description

La présente invention a pour objet un procédé d'acylation d'un éther aromatique.

L'invention vise préférentiellement un procédé d'acylation d'un éther aromatique non substitué et plus particulièrement de l'anisole.

L'invention s'applique à la préparation d'alkylcétones alkoxyaromatiques.

Les procédés classiques d'acylation des composés aromatiques, notamment des éthers de phénols consistent à effectuer une réaction d'acylation de type Friedel-Crafts.

On fait réagir le composé aromatique et un agent d'acylation, en présence d'un catalyseur qui est généralement le chlorure d'aluminium.

Une illustration de ce type de procédé est donnée par les travaux de C. KURODA et coll. [Sci. Papers Inst. Phys. Chem. Res. 18, pp. 51-60 (1932)] qui ont décrit la préparation de méthoxyacétophénones, par réaction d'un composé aromatique porteur de 1 à 3 groupes méthoxy, avec du chlorure d'acétyle, en présence de chlorure d'aluminium.

Toutefois, la mise en oeuvre du chlorure d'aluminium présente de nombreux inconvénients. Le chlorure d'aluminium est un produit corrosif et irritant. De plus, il est nécessaire de mettre en oeuvre une quantité importante de chlorure d'aluminium au moins égale à la stoechiométrie, par suite de la complexation de la cétone formée. En conséquence, le chlorure d'aluminium n'est donc pas un vrai catalyseur.

En fin de réaction, il est nécessaire d'éliminer le chlorure d'aluminium du milieu réactionnel en faisant une hydrolyse acide ou basique.

Cette technique d'hydrolyse implique l'addition d'eau dans le milieu réactionnel ce qui complique notablement la mise en oeuvre du procédé car le cation métallique, et plus particulièrement le cation aluminium forme alors en présence d'eau, des complexes polyoxo- et/ou polyhydroxo- d'aluminium de consistance laiteuse, difficiles ensuite à séparer. Il en résulte la nécessité de faire un traitement long et coûteux comportant après l'hydrolyse, une extraction de la phase organique, une séparation des phases aqueuse et organique, voire-même un séchage de cette dernière. La séparation du chlorure d'aluminium est donc longue et coûteuse.

Par ailleurs, se pose le problème des effluents aqueux salins qu'il faut ensuite neutraliser ce qui impose une opération supplémentaire.

De plus, le chlorure d'aluminium ne peut être recyclé du fait de son hydrolyse.

Pour pallier cet inconvénient, on a proposé de faire la réaction en présence de catalyseurs hétérogènes.

Ainsi, depuis une dizaine d'années, on a préconisé de mettre en oeuvre, les zéolithes, comme catalyseurs d'acylation.

Prins et coll. ont décrit l'acétylation de l'anisole, par l'anhydride acétique [9$^{th}$ International Zeolite Congress - Montréal Congrès (1992)], en présence de zéolithes telles que zéolithe β ou zéolithe USY. Il est à noter que les zéolithes β permettent d'obtenir des résultats plus intéressants tant en ce qui concerne le taux de conversion que le rendement réactionnel.

Toutefois, les performances du catalyseur décrit ne donnent pas satisfaction. La mise en oeuvre dudit catalyseur à l'échelle industrielle pose un problème car la productivité du catalyseur est insuffisante si bien qu'il serait nécessaire de prévoir une taille de réacteur très conséquente.

L'objet de la présente invention est de fournir un procédé permettant d'obvier aux inconvénients précités.

Il a maintenant été trouvé et c'est ce qui constitue l'objet de la présente invention, un procédé d'acylation d'un éther aromatique qui consiste à faire réagir ledit éther avec un agent d'acylation, en présence d'un catalyseur zéolithique caractérisé par le fait que l'on conduit la réaction d'acylation en faisant réagir l'éther aromatique et un agent d'acylation choisi parmi les halogénures et/ou les anhydrides d'acides carboxyliques aliphatiques ou cycloaliphatiques, en présence d'une quantité efficace d'un catalyseur comprenant une zéolithe bêta présentant un rapport atomique dénommé "Si/$Me^1$ global", entre le nombre d'atomes de l'élément silicium et le nombre d'atomes de tout élément trivalent $Me^1$ contenu dans la zéolithe, supérieur ou égal à 15, de préférence compris entre 15 et 55, et de manière encore plus préférée entre 18 et 35.

Par "$Me^1$", on désigne tout élément ayant un degré d'oxydation de +3 et notamment, l'aluminium, la gallium, le fer, le bore et leurs mélanges, et de préférence, l'aluminium.

Le rapport atomique "Si/$Me^1$ global" de la zéolithe est déterminé notamment par fluorescence X.

Le catalyseur mis en oeuvre dans le procédé de l'invention comprend une phase active qui est une zéolithe bêta (β) de type structural BEA ayant certaines caractéristiques bien définies. Ainsi, elle présente une faible teneur en élément $Me^1$, qui est de préférence, l'aluminium.

A l'état déshydraté, la zéolithe a, plus précisément, une composition chimique correspondant à la formule empirique suivante :

$$(1\text{-}x)H_2O,\ x\ [(Me^2)_2O],\ [(Me^1)_2O_3],\ 2n\ SiO_2\ (F)$$

dans ladite formule :

- $Me^1$ représente tout élément ayant un degré d'oxydation de +3, de préférence, l'aluminium, la gallium, le fer, le bore et leurs mélanges,
- $Me^2$ représente un métal choisi dans le groupe des éléments de la colonne 1a et leurs mélanges, de préférence les métaux alcalins tels que le lithium, le sodium, le potassium, le rubidium et le césium,
- n, égal au rapport atomique "Si/$Me^1$ global" défini précédemment, est compris entre 15 et 55, de préférence entre 18 et 35,
- x, égal au rapport atomique $Me^2/Me^1$, entre le nombre d'atomes de métal alcalin $Me^2$ et le nombre d'atomes de tout élément trivalent $Me^1$ de la zéolithe, est compris entre 0 et 0,2, de préférence entre 0,005 et 0,1.

Par "$Me^2$", on entend un métal choisi dans le groupe des éléments de la colonne 1a et leurs mélanges, de préférence les métaux alcalins tels que le lithium, le sodium, le potassium, le rubidium et le césium. $Me^2$ représente de préférence, le sodium ou le potassium

Pour la définition des éléments, on se réfère ci-après à la Classification périodique des éléments publiée dans le Bulletin de la Société Chimique de France, n°1 (1966).

Les zéolithes bêta sont décrites dans la littérature [cf. Atlas of zeolites structure types by W. M. Meier and D. H. Olson published by the Structure Commission of the International Zeolite Association (1978) ou brevet US 3 308 069 ou article Caullet P. et coll., Zeolites 12, pp. 240 (1992)].

Elles peuvent être synthétisées en milieu basique classique ou bien en milieu fluorure conformément à FR-A 2 653 347.

Afin de mettre en oeuvre une zéolithe β répondant aux caractéristiques précitées, il peut être nécessaire d'effectuer un traitement de désalumination de la zéolithe de telle sorte que le rapport atomique "Si/$Me^1$ global" défini précédemment soit compris dans l'intervalle précité.

Ainsi, on peut mettre en oeuvre les méthodes connues de l'homme du métier parmi lesquelles on peut citer, à titre d'exemples, et de manière non exhaustive, les calcinations en présence de vapeur, les calcinations en présence de vapeur d'eau suivies d'attaques par des acides minéraux ($HNO_3$, HCl...), les traitements directes de désalumination par des réactifs tels que le tétrachlorure de silicium ($SiCl_4$), l'hexafluorosilicate d'ammonium ($(NH_4)_2SiF_6$), l'éthylènediaminetétracétique (EDTA) ainsi que sa forme mono- ou disodique. On peut également faire un traitement de désalumination par attaque acide directe par des solutions d'acides minéraux tels que par exemple, l'acide chlorhydrique, l'acide nitrique, l'acide sulfurique ou d'acides organiques comme notamment l'acide acétique, l'acide oxalique.

Par ailleurs, toute combinaison des méthodes de désalumination précitées est aussi possible.

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Comme mentionné précédemment, le procédé de l'invention convient pour effectuer une réaction d'acylation sur un éther aromatique, de préférence, non substitué.

Dans l'exposé qui suit de la présente invention, on désigne par "éther aromatique", un composé aromatique dont un atome d'hydrogène directement lié au noyau aromatique est remplacé par un groupe éther et par "aromatique", la notion classique d'aromaticité telle que définie dans la littérature, notamment par Jerry MARCH, Advanced Organic Chemistry, 4ème édition, John Wiley and Sons, 1992, pp. 40 et suivantes.

On entend par "éther aromatique non substitué", un éther aromatique ne comprenant pas d'autres substituants sur le noyau aromatique.

Plus précisément, la présente invention a pour objet un procédé d'acylation d'un éther aromatique de formule générale (I):

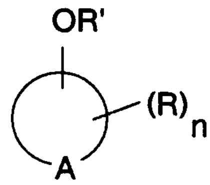

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

Dans le présent texte, on désigne, de manière simplifiée, par "groupes alkoxy", les groupes du type R'-O- dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant cyclique.

L'éther aromatique qui intervient dans le procédé de l'invention répond à la formule (I) dans laquelle R' représente un radical aliphatique acyclique, saturé ou insaturé, linéaire ou ramifié.

Plus préférentiellement, R' représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène).

Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Le radical aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux éthers aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle et éthyle.

Dans la formule générale (I) des éthers aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés. On peut citer plus particulièrement, un reste naphtalénique.

Le reste A peut porter un ou plusieurs substituants sur le noyau aromatique.

Des exemples de substituants R sont donnés ci-après mais cette liste ne présente pas de caractère limitatif. N'importe quel substituant peut être présent sur le cycle dans la mesure où il n'interfère pas au niveau du produit désiré.

Le procédé de l'invention s'applique plus particulièrement, aux éthers aromatiques de formule (Ia):

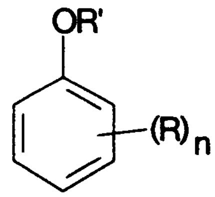

(Ia)

dans laquelle :

- n est un nombre inférieur ou égal à 4, de préférence égal à 0 ou 1,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle,
- le ou les radicaux R représentent l'un des atomes ou groupes suivants :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical cyclohexyle ou benzyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

    - $R_1$-OH
    - $R_1$-$COOR_2$
    - $R_1$-CHO
    - $R_1$-$NO_2$
    - $R_1$-CN
    - $R_1$-N-$(R_2)_2$
    - $R_1$-CO-N-$(R_2)_2$
    - $R_1$-X
    - $R_1$-$CF_3$

  dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux OR' et R peuvent représenter un radical méthylène dioxy ou éthylène dioxy.
Dans la formule (Ia), R' représente préférentiellement un radical alkyle, linéaire ou ramifié, ayant de 1 à 4 atomes de carbone, de préférence, un radical méthyle ou éthyle.
L'éther aromatique de formule (I) peut porter un ou plusieurs substituants R.
R représente plus préférentiellement, l'un des atomes ou groupes suivants :

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un atome d'halogène, de préférence un atome de fluor, chlore ou brome, un radical trifluorométhyle.

Dans la formule (Ia), R représente préférentiellement un radical alkoxy, linéaire ou ramifié, ayant de 1 à 4 atomes de carbone, de préférence, un radical méthoxy ou éthoxy.

Interviennent préférentiellement dans le procédé de l'invention des éthers aromatiques de formule (I) ou (Ia) dans lesquelles :

- n est égal à 0 ou 1,
- R' représente un radical alkyle, linéaire ou ramifié, ayant de 1 à 6 atomes de carbone ou un radical phényle,
- R représente un radical alkoxy, linéaire ou ramifié, ayant de 1 à 4 atomes de carbone, de préférence, un radical méthoxy ou éthoxy.
- les radicaux OR' et R forment un radical méthylène dioxy ou éthylène dioxy.

Le procédé de l'invention s'applique plus particulièrement, aux éthers aromatiques non substitués c'est-à-dire à des éthers aromatiques de formule (I) ou (Ia) dans lesquelles n est égal à 0 : R' représentant de préférence, un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle.

Il convient tout à fait bien pour les éthers aromatiques de formule (Ia) dans laquelle le radical R' est un radical alkyle, de préférence, méthyle ou éthyle.

A titre illustratif de composés répondant à la formule (I), on peut mentionner plus particulièrement :

- les monoéthers non substitués tels que l'anisole, l'éthoxybenzène (phénétole), le propoxybenzène, l'isopropoxybenzène, le butoxybenzène, l'isobutoxybenzène, le 1-méthoxynaphtalène, le 2-méthoxynaphtalène, le 2-éthoxynaphtalène ; les monoéthers substitués tels que le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-benzylanisole, le 2-cyclohexylanisole, le 1-bromo-2-éthoxybenzène, le 1-bromo-3-éthoxybenzène, le 1-chloro-2-éthoxybenzène, le 1-chloro-3-éthoxybenzène, le 1-éthoxy-2-éthylbenzène, le 1-éthoxy-3-éthylbenzène, le 1-méthoxy-2-allyloxybenzène, le 2,3-diméthylanisole, le 2,5-diméthylanisole,
- les diéthers comme le vératrole, le 1,3-diméthoxybenzène, le 1,4-diméthoxybenzène, le 1,2-diéthoxy- benzène, le 1,3-diéthoxybenzène, le 1,2-dipropoxybenzène, le 1,3-dipropoxy- benzène, le 1,2-méthylènedioxybenzène, le 1,2-éthylènedioxybenzène,
- les triéthers comme le 1,2,3-triméthoxybenzène, le 1,3,5-triméthoxybenzène, le 1,3,5-triéthoxybenzène.

Les composés auxquels s'applique de manière plus particulièrement intéressante le procédé selon l'invention, sont les éthers non substitués répondant préférentiellement à la formule (I) ou (Ia) dans lesquelles n est égal à 0. L'invention est bien adaptée pour effectuer l'acylation de l'anisole.

Pour ce qui est du réactif d'acylation, il est choisi dans le groupe formé par les halogénures d'acides carboxyliques et les anhydrides d'acides carboxyliques.

Les acides carboxyliques sont des acides carboxyliques aliphatiques, saturés ou insaturés, linéaires ou ramifiés ou des acides cycloaliphatiques, éventuellement substitués, saturés ou insaturés.

Ils répondent plus particulièrement à la formule (II) :

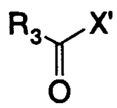
**(II)**

dans laquelle :

- $R_3$ représente :
  - . un radical aliphatique saturé ou insaturé, linéaire ou ramifié ayant de 1 à 24 atomes de carbone ; un radical cycloaliphatique, saturé ou insaturé, monocyclique ou polycyclique, ayant de 3 à 12 atomes de carbone ;
- X' représente :

- . un atome d'halogène, de préférence un atome de chlore ou de brome,
- . un radical -O-CO-$R_4$ avec $R_4$, identique ou différent de $R_3$, ayant la même signification que $R_3$ : $R_3$ et $R_4$ pouvant former ensemble un radical divalent aliphatique saturé ou insaturé, linéaire ou ramifié, ayant au moins 2 atomes de carbone.

Plus préférentiellement, $R_3$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse de substituants (par exemple, des atomes d'halogène ou un groupe $CF_3$).

$R_3$ représente également un radical alcényle ayant de 2 à 10 atomes de carbone, tel que vinyle, propèn-yle, butèn-yle, pentèn-yle, hexèn-yle, octèn-yle, décèn-yle.

Le radical $R_3$ représente également un radical non aromatique, de préférence, cycloaliphatique, par exemple, un radical cyclohexyle, qui peut être éventuellement substitué. N'importe quel substituant peut être présent sur le cycle dans la mesure où il n'interfère pas au niveau du produit souhaité.

Comme exemples plus particuliers de substituants, on peut citer, notamment :

- . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
- . un groupe hydroxyle,
- . un atome d'halogène, de préférence un atome de fluor, chlore ou brome.

Les agents d'acylation préférés sont les anhydrides d'acides. Ils répondent plus particulièrement à la formule (II) dans laquelle $R_3$ et $R_4$ sont identiques et représentent un radical alkyle ayant de 1 à 4 atomes de carbone, éventuellement porteurs d'atomes d'halogène, de préférence, de chlore.

Lorsque l'agent d'acylation est un halogénure d'acide, il répond préférentiellement à la formule (II) dans laquelle X' représente un atome de chlore et $R_3$ représente un radical alkyle ayant de 1 à 4 atomes de carbone, de préférence, méthyle ou éthyle éventuellement porteurs d'atomes d'halogène, de préférence, de chlore.

A titre illustratif d'agents d'acylation répondant à la formule (II), on peut citer plus particulièrement :

- l'anhydride acétique,
- l'anhydride propanoïque,
- l'anhydride isobutyrique,
- l'anhydride trifluoroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

Conformément à l'invention, la réaction d'acylation est conduite avantageusement en phase liquide comprenant l'éther aromatique et l'agent d'acylation et en présence du catalyseur.

L'un des réactifs de départ peut servir de solvant réactionnel mais il est également possible de faire appel à un solvant organique.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques ou des solvants aprotiques, plus polaires.

A titre d'exemples d'hydrocarbures aliphatiques ou cycloaliphatiques, on peut citer plus particulièrement les paraffines tels que notamment, l'hexane, l'heptane, l'octane, le nonane, le décane, le undécane, le dodécane, le tétradécane ou le cyclohexane, et les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachloroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs dibromobenzènes ; le 1-bromonaphtalène.

On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy 3-oxapentane) ; l'oxyde de biphényle ou de benzyle ; le dioxane, le tétrahydrofuranne (THF).

Comme exemples de solvants organiques aprotiques, plus polaires qui peuvent également être mis en oeuvre dans le procédé de l'invention, on peut citer plus particulièrement les carboxamides linéaires ou cycliques comme le N,N-diméthylacétamide (DMAC), le N,N-diéthylacétamide, le diméthylformamide (DMF), le diéthylformamide ou la 1-méthyl 2-pyrrolidinone (NMP) ; le diméthylsulfoxyde (DMSO) ; les sulfones cycliques ou non telles que la tétraméthylsulfone, la diméthylsulfone ; l'hexaméthylphosphotriamide (HMPT) ; les urées tétrasubstituées cycliques ou non comme la diméthyléthylèneurée, la diméthylpropylèneurée, la tétraméthylurée.

Les solvants préférés sont : le dichlorométhane, le tétrachlorométhane, le THF et l'oxyde de diéthyle.

On peut également utiliser un mélange de solvants organiques.

On utilise préférentiellement, le substrat de départ comme solvant réactionnel.

Comme mentionné précédemment, l'éther aromatique est mis à réagir avec un agent d'acylation, éventuellement en présence d'un solvant réactionnel tel que défini et en présence d'un catalyseur solide défini ci-avant.

Le rapport entre le nombre de moles d'éther aromatique et le nombre de moles d'agent d'acylation peut varier car le substrat peut servir de solvant réactionnel. Ainsi, le rapport peut aller de 0,1 à 20, et se situe de préférence entre 0,5 et 10.

La quantité de catalyseur que l'on met en oeuvre dans le procédé de l'invention peut varier dans de larges limites.

Lorsque l'on réalise le procédé en discontinu, le catalyseur peut représenter en poids par rapport à l'éther aromatique engagé, de 0,01 à 50 %, de préférence, de 1,0 à 20 %. Cependant si l'on réalise le procédé en continu, par exemple en faisant réagir un mélange de l'éther aromatique et de l'agent d'acylation sur un lit fixe de catalyseur, ces rapports catalyseur/éther aromatique n'ont pas de sens et à un instant donné, on pourra avoir un excès pondéral de catalyseur par rapport à l'éther aromatique de départ.

Pour ce qui est de la quantité de solvant organique mis en oeuvre, elle est choisie généralement de telle sorte que le rapport entre le nombre de moles de solvant organique et le nombre de moles d'éther aromatique varie, de préférence, entre 0 et 100 et encore plus préférentiellement entre 0 et 50.

Il est également possible de conduire le procédé de l'invention, en présence d'eau : cette dernière pouvant représenter de 0 à 10 % du poids de l'agent d'acylation.

La température à laquelle est mise en oeuvre la réaction d'acylation dépend de la réactivité du substrat de départ et de celle de l'agent d'acylation.

Elle se situe entre 20°C et 300°C, de préférence entre 40°C et 200°C.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

D'un point de vue pratique, le procédé peut être mis en oeuvre en discontinu ou en continu.

Selon la première variante, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

L'autre variante de l'invention consiste à conduire la réaction en continu, dans un réacteur tubulaire comportant le catalyseur solide disposé en lit fixe.

L'éther aromatique et l'agent d'acylation peuvent être introduits séparément ou en mélange dans le réacteur.

Ils peuvent également être introduits dans un solvant tel que mentionné précédemment.

Le temps de séjour du flux de matière sur le lit catalytique varie, par exemple, entre 15 mn et 10 heures, et de préférence, entre 30 mn et 5 heures.

En fin de réaction, on obtient une phase liquide comprenant l'éther aromatique acylé qui peut être récupéré de manière classique, par distillation ou par recristallisation dans un solvant approprié, après élimination préalable des réactifs en excès.

Le procédé de l'invention est particulièrement bien adapté à la préparation de la 4-méthoxyacétophénone dénommée couramment acétoanisole, par acétylation de l'anisole.

Un avantage du procédé de l'invention est que la réaction d'acylation s'effectue sans qu'il y ait une O-désalkylation de l'éther aromatique de départ.

Les exemples qui suivent, illustrent l'invention sans toutefois la limiter.

Les exemples 1 et 2 se rapportent à la préparation des zéolithes qui sont mis en oeuvre dans les exemples d'application 3, 4, 6 à 8.

Les exemples 5 et 9 sont donnés à titre comparatif.

Dans les exemples, les rendements mentionnés correspondent à la définition suivante :

$$\text{Rendement : } RR_{A.A.} = \frac{\text{nombre de moles de composé aromatique acylé formées}}{\text{nombre de moles d'agent d'acylation introduites}}\ \%$$

Exemple 1

Dans cet exemple, on prépare une zéolithe β dont le rapport "Si/Al global" est de 25,2.

La zéolithe de départ est une zéolithe de rapport "Si/Al global" de 12,5 commercialisée par la Société PQ Zeolites sous la référence CVB 811 BL25.

Dans un ballon muni d'un réfrigérant, on met en suspension 50 g de ladite zéolithe β, dans 250 ml d'une solution d'acide nitrique 0,5 N.

On porte le mélange à reflux durant 3 heures.

Puis on lave la zéolithe à l'eau distillée jusqu'à ce que le pH des eaux de lavage devienne neutre.

Le rapport "Si/Al global", déterminé par fluorescence X, de la zéolithe β ainsi préparée est de 25,2.

Exemple 2

Dans cet exemple, on prépare une zéolithe β dont le rapport "Si/Al global" est de 43.

Dans un ballon muni d'un réfrigérant, on met en suspension 50 g de zéolithe β définie dans l'exemple 1 de rapport "Si/Al global" égal à 12,5, dans 250 ml d'une solution d'acide nitrique 1,3 N.

On porte le mélange à reflux durant 4 heures.

Puis on lave la zéolithe à l'eau distillée jusqu'à ce que le pH des eaux de lavage devienne neutre.

Le rapport "Si/Al global", déterminé par fluorescence X, de la zéolithe β ainsi préparée est de 43.

Exemple 3

Cet exemple d'application concerne l'utilisation de la zéolithe préparée selon l'exemple 1 pour effectuer l'acétylation de l'anisole.

Dans un réacteur tubulaire, chauffé par une double enveloppe, on introduit 13 ml de zéolithe β (soit environ 5 g) sous forme de poudre.

La double enveloppe est chauffée à 100°C et l'on introduit alors par le bas du réacteur à l'aide d'une pompe HPLC un mélange d'anisole et d'anhydride acétique dans un rapport molaire de 2, avec un débit de 0,2 ml/min.

On soutire le mélange réactionnel en continu par débordement.

On suit le rendement de la réaction au cours du temps par prélèvement d'aliquotes qui sont ensuite analysés par chromatographie en phase gazeuse.

Les résultats obtenus sont les suivants :

Tableau (I)

| Temps (heures) | $RR_{A.A.}$ (%) |
|---|---|
| 0 | 85 |
| 1 | 98 |
| 6 | 100 |
| 22 | 90 |
| 25 | 89 |
| 30 | 85 |

On peut ainsi après 30 heures de fonctionnement isoler 306 g d'acétoanisole. Dans ces conditions, pour une

durée de 30 heures, la productivité exprimée en gramme d'acétoanisole par gramme de catalyseur et par heure est de : 2,04 g.

Exemple 4

Cet exemple d'application concerne l'utilisation de la zéolithe préparée selon l'exemple 2 pour effectuer l'acétylation de l'anisole.
Dans un réacteur tubulaire, chauffé par une double enveloppe, on introduit 13 ml de zéolithe β (soit environ 5 g) sous forme de poudre.
La double enveloppe est chauffée à 100°C et l'on introduit alors par le bas du réacteur à l'aide d'une pompe HPLC un mélange d'anisole et d'anhydride acétique dans un rapport molaire de 2, avec un débit de 0,2 ml/min.
On soutire le mélange réactionnel en continu par débordement.
On suit le rendement de la réaction au cours du temps par prélèvement d'aliquotes qui sont ensuite analysés par chromatographie en phase gazeuse.
Les résultats obtenus sont les suivants :

Tableau (II)

| Temps (heures) | $RR_{A.A.}$ (%) |
|---|---|
| 0 | 51,3 |
| 6 | 93,5 |
| 23 | 78 |
| 30 | 75 |

On peut ainsi après 30 heures de fonctionnement isoler 229 g d'acétoanisole. Dans ces conditions, pour une durée de 30 heures, la productivité est de 1,52 g par gramme de catalyseur et par heure.

Exemple 5*

Dans cet essai comparatif, on met en oeuvre la zéolithe décrite par Prins et colt. (op. cit.), pour effectuer l'acétylation de l'anisole.
La zéolithe utilisée est la zéolithe de rapport "Si/Al global" de 12,5 commercialisée par la Société PQ Zeolites sous la référence CVB 811 BL25.
Dans un réacteur tubulaire, chauffé par une double enveloppe, on introduit 13 ml de zéolithe β (soit environ 5,1 g) sous forme de poudre.
La double enveloppe est chauffée à 100°C et l'on introduit alors par le bas du réacteur à l'aide d'une pompe HPLC un mélange d'anisole et d'anhydride acétique dans un rapport molaire de 2, avec un débit de 0,2 ml/min.
On soutire le mélange réactionnel en continu par débordement.
On suit le rendement de la réaction au cours du temps par prélèvement d'aliquotes qui sont ensuite analysés par chromatographie en phase gazeuse.
Les résultats obtenus sont les suivants :

Tableau (III)

| Temps (heures) | $RR_{A.A.}$ (%) |
|---|---|
| 0 | 60 |
| 1 | 54 |
| 2,5 | 54 |
| 3,5 | 51,6 |
| 4,5 | 53,4 |
| 21,25 | 41 |
| 26,75 | 41 |
| 28,75 | 38 |

On peut ainsi après 30 heures de fonctionnement isoler 83 g d'acétoanisole.
Dans ces conditions, pour une durée de 30 heures, la productivité est de 0.54 g par gramme de catalyseur et par heure.

Exemple 6

Acétylation du vératrole

Dans un réacteur fermé de 300 ml, on charge 119,5 g de vératrole (0,86 mol), 43,6 g d'anhydride acétique (0,43 mol) et 11,2 g de catalyseur préparé selon l'exemple 1.
Le réacteur est alors chauffé à 90°C.
Après 7 heures, le mélange réactionnel est filtré puis analysé par chromatrographie en phase gazeuse.
Le rendement en acétovératrole (ou 3,4-diméthoxyacétophénone) obtenu est :

$RR_{AA}$ = 82 %.

Exemple 7

Acétylation du vératrole

L'acétylation du vératrole est réalisée dans les conditions opératoires de l'exemple 6, avec le catalyseur de l'exemple 2.
Après 7 heures, le rendement en acétovératrole obtenu est :

$RR_{AA}$ = 78 %.

Exemple 8

Acétylation du 1,2-méthylènedioxybenzène

On réalise l'acétylation du 1,2-méthylènedioxybenzène selon les conditions décrites dans l'exemple 6 mais avec le catalyseur décrit dans l'exemple 1.
On charge 119 g de 1,2-méthylènedioxybenzène (0,97 mol), 50,4g d'anhydride acétique (0,49 mol) et 12,2 g de catalyseur.
Après 7 heures, le rendement en produit acétylé en para obtenu est :

$RR_{AA}$ = 61 %.

Exemple 9*

Dans cet essai comparatif, on effectue l'acétylation du 1,2-méthylènedioxybenzène comme dans l'exemple 8 mais en mettant en oeuvre la zéolithe décrite par Prins et coll. (op. cit.).
Après 7 heures, le rendement en produit acétylé en para obtenu est :

$RR_{AA}$ = 37 %.

## Revendications

1. Procédé d'acylation d'un éther aromatique qui consiste à faire réagir ledit éther avec un agent d'acylation, en présence d'un catalyseur zéolithique caractérisé par le fait que l'on conduit la réaction d'acylation en faisant réagir l'éther aromatique et un agent d'acylation choisi parmi les halogénures et/ou les anhydrides d'acides carboxyliques aliphatiques ou cycloaliphatiques, en présence d'une quantité efficace d'un catalyseur comprenant une zéolithe bêta présentant un rapport atomique dénommé "Si/$Me^1$ global", entre le nombre d'atomes de l'élément silicium et le nombre d'atomes de tout élément trivalent $Me^1$ contenu dans la zéolithe, supérieur ou égal à 15, de préférence compris entre 15 et 55, et de manière encore plus préférée entre 18 et 35.

2. Procédé selon la revendication 1 caractérisé par le fait que la zéolithe de type bêta présente un rapport atomique $Me^2/Me^1$, entre le nombre d'atomes de métal alcalin $Me^2$ et le nombre d'atomes de tout élément trivalent $Me^1$ de la zéolithe, compris entre 0 et 0,2, de préférence entre 0,005 et 0,1.

3. Procédé selon la revendication 1 caractérisé par le fait que l'éther aromatique répond à la formule générale (I):

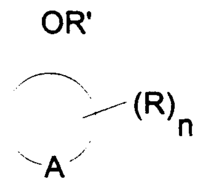

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

**4.** Procédé selon la revendication 3 caractérisé par le fait que l'éther aromatique répond à la formule générale (I) dans laquelle R' représente :

- un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
- un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
- un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué.
- un radical carbocyclique aromatique, de préférence monocyclique ayant généralement au moins 4 atomes de carbone, de préférence, 6 atomes de carbone dans le cycle ; ledit cycle pouvant être substitué.

**5.** Procédé selon la revendication 3 caractérisé par le fait que l'éther aromatique répond à la formule générale (I) dans laquelle R' représente un radical alkyle linéaire ou ramifié ayant de 1 à 4 atomes de carbone, de préférence un radical méthyle ou un radical phényle ou dans laquelle les radicaux R' et R forment un radical méthylène dioxy ou éthylène dioxy.

**6.** Procédé selon l'une des revendications 3 à 5 caractérisé par le fait que l'éther aromatique répond à la formule générale (I) dans laquelle le reste A représente le reste d'un composé carbocyclique aromatique, monocyclique ayant au moins 4 atomes de carbone et de préférence 6 atomes de carbone ou le reste d'un composé carbocyclique polycyclique, de préférence un reste naphtalénique : le reste A pouvant porter un ou plusieurs substituants sur le noyau aromatique.

**7.** Procédé selon la revendication 1 caractérisé par le fait que l'éther aromatique répond à la formule (Ia):

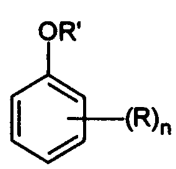

(Ia)

dans laquelle :

- n est un nombre inférieur ou égal à 4, de préférence égal à 0 ou 1,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle,
- le ou les radicaux R représentent l'un des atomes ou groupes suivants :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical cyclohexyle ou benzyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

    - $R_1$-OH
    - $R_1$-$COOR_2$
    - $R_1$-CHO
    - $R_1$-$NO_2$
    - $R_1$-CN
    - $R_1$-N-$(R_2)_2$
    - $R_1$-CO-N-$(R_2)_2$
    - $R_1$-X
    - $R_1$-$CF_3$

  dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

**8.** Procédé selon la revendication 7 caractérisé par le fait que l'éther aromatique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence l'oxygène : les radicaux OR' et R formant de préférence un radical méthylène dioxy ou éthylène dioxy.

**9.** Procédé selon les revendications 3 et 7 caractérisé par le fait que l'éther aromatique est un éther aromatique répondant à la formule (I) ou (Ia) dans lesquelles :

- n est égal à 0 ou 1,
- R' représente un radical alkyle, linéaire ou ramifié, ayant de 1 à 6 atomes de carbone ou un radical phényle,
- R représente un radical alkoxy, linéaire ou ramifié, ayant de 1 à 4 atomes de carbone, de préférence, un radical méthoxy ou éthoxy.
- les radicaux OR' et R forment un radical méthylène dioxy ou éthylène dioxy.

**10.** Procédé selon les revendications 3 et 7 caractérisé par le fait que l'éther aromatique est un éther aromatique non substitué répondant à la formule (I) ou (Ia) dans lesquelles n est égal à 0 ; le radical R' représente de préférence, un radical alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle , ledit éther étant de préférence, l'anisole.

**11.** Procédé selon l'une des revendications 1 à 10 caractérisé par le fait que l'agent d'acylation répond à la formule (II) :

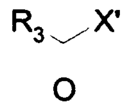

(II)

dans laquelle :

- $R_3$ représente :
  - . un radical aliphatique saturé ou insaturé, linéaire ou ramifié ayant de 1 à 24 atomes de carbone ; un radical cycloaliphatique, saturé ou insaturé, monocyclique ou polycyclique, ayant de 3 à 12 atomes de carbone ;
- X' représente :
  - . un atome d'halogène, de préférence un atome de chlore ou de brome,
  - . un radical $-O-CO-R_4$ avec $R_4$, identique ou différent de $R_3$, ayant la même signification que $R_3$ : $R_3$ et $R_4$ pouvant former ensemble un radical divalent aliphatique saturé ou insaturé, linéaire ou ramifié, ayant au moins 2 atomes de carbone.

**12.** Procédé selon la revendication 11 caractérisé par le fait que l'agent d'acylation répond à la formule (II) dans laquelle X' représente un atome de chlore et $R_3$ représente un radical alkyle linéaire ou ramifié ayant de 1 à 12 atomes de carbone, de préférence, 1 à 4 atomes : la chaîne hydrocarbonée pouvant être éventuellement interrompue par un hétéroatome ou par un groupe fonctionnel ou porteuse de substituants, de préférence, d'atomes d'halogène ; X' représente un radical $-O-CO-R_4$. dans laquelle $R_3$ et $R_4$ sont identiques et représentent un radical alkyle ayant de 1 à 4 atomes de carbone éventuellement porteur d'atomes d'halogène.

**13.** Procédé selon la revendication 11 et 12 caractérisé par le fait que l'agent d'acylation est choisi parmi :

- l'anhydride acétique,
- l'anhydride propanoïque,
- l'anhydride isobutyrique,
- l'anhydride trifluoroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

**14.** Procédé selon l'une des revendications 1 à 13 caractérisé par le fait que le catalyseur est une zéolithe bêta ayant, à l'état déshydraté, la composition chimique correspondant à la formule empirique suivante :

$$(1-x)H_2O,\ x[(Me^2)_2O],\ [(Me^1)_2O_3],\ 2n\ SiO_2 \quad (F)$$

dans ladite formule :

- $Me^1$ représente tout élément ayant un degré d'oxydation de +3, de préférence, l'aluminium, la gallium, le fer, le bore et leurs mélanges,
- $Me^2$ représente un métal choisi dans le groupe des éléments de la colonne 1a et leurs mélanges, de préférence les métaux alcalins tels que le lithium, le sodium, le potassium, le rubidium et le césium,
- n, égal au rapport atomique "Si/$Me^1$ global" défini précédemment, est compris entre 15 et 55, de préférence entre 18 et 35,

- x, égal au rapport atomique $Me^2/Me^1$, entre le nombre d'atomes de métal alcalin $Me^2$ et le nombre d'atomes de tout élément trivalent $Me^1$ de la zéolithe, est compris entre 0 et 0,2, de préférence entre 0,005 et 0,1.

**15.** Procédé selon la revendication 14 caractérisé par le fait que le catalyseur est une zéolithe répondant à la formule chimique (F) dans laquelle $Me^1$ représente l'aluminium et $Me^2$ le sodium et/ou le potassium.

**16.** Procédé selon l'une des revendications 1 à 15 caractérisé par le fait que une zéolithe bêta est soumise à un traitement de désalumination de telle sorte que le rapport atomique "Si/$Me^1$ global" défini précédemment soit compris dans l'intervalle précité.

**17.** Procédé selon la revendication 16 caractérisé par le fait que les traitements de désalumination sont les calcinations en présence de vapeur, les calcinations en présence de vapeur d'eau suivies d'attaques par des acides minéraux ($HNO_3$, HCl...), les traitements de désalumination par des réactifs tels que le tétrachlorure de silicium ($SiCl_4$), l'hexafluorosilicate d'ammonium ($(NH_4)_2SiF_6$) ou bien encore l'éthylènediaminetétracétique (EDTA) ainsi que sa forme mono ou disodique ; l'attaque acide directe par des solutions d'acides minéraux ou d'acides organiques ou toute combinaison desdites méthodes.

**18.** Procédé selon l'une des revendications 1 à 17 caractérisé par le fait que la zéolithe est mise en oeuvre seule ou en mélange avec une matrice minérale choisie, de préférence, parmi les oxydes de métaux, tels que les oxydes d'aluminium, de silicium et/ou de zirconium, ou encore parmi les argiles et plus particulièrement, le kaolin, le talc ou la montmorillonite.

**19.** Procédé selon l'une des revendications 1 à 18 caractérisé par le fait que l'on met en oeuvre un solvant organique choisi parmi :

- les hydrocarbures aliphatiques, cycloaliphatiques ou aromatiques.
- les hydrocarbures halogénés aliphatiques ou aromatiques,
- les éther-oxydes aliphatiques, cycloaliphatiques ou aromatiques,
- les carboxamides linéaires ou cycliques,
- le diméthylsulfoxyde (DMSO),
- les sulfones cycliques ou non,
- l'hexaméthylphosphotriamide (HMPT),
- les urées tétrasubstituées cycliques ou non.

**20.** Procédé selon l'une des revendications 1 à 19 caractérisé par le fait que le rapport entre le nombre de moles d'éther aromatique et le nombre de moles d'agent d'acylation varie entre 0,1 et 20, et se situe de préférence entre 0,5 et 10.

**21.** Procédé selon l'une des revendications 1 à 20 caractérisé par le fait que la quantité de catalyseur représente en poids par rapport à l'éther aromatique engagé, de 0,01 à 50 %, de préférence, de 1,0 à 20 %.

**22.** Procédé selon l'une des revendications 1 à 21 caractérisé par le fait que la température à laquelle est mise en oeuvre la réaction d'acylation se situe entre 20°C et 300°C, de préférence entre 40°C et 200°C.

**Patentansprüche**

**1.** Verfahren zur Acylierung eines aromatischen Ethers, das darin besteht, den genannten Ether mit einem Acylierungsmittel in Gegenwart eines zeolithischen Katalysators reagieren zu lassen, dadurch gekennzeichnet, daß man die Acylierungsreaktion ausführt, indem man den aromatischen Ether und ein Acylierungsmittel, das aus den Halogeniden und/oder den Anhydriden von aliphatischen oder cycloaliphatischen Carboxylsäuren gewählt ist, in Gegenwart einer wirksamen Menge eines Katalysators reagieren läßt, der einen Beta-Zeolith enthält, dessen Atomverhältnis, genannt "Si/$Me^1$ global", zwischen der Anzahl der Atome des Elements Silicium und der Anzahl der Atome aller im Zeolith enthaltenen dreiwertigen Elemente $Me^1$ größer oder gleich 15 ist, vorzugsweise zwischen 15 und 55 und insbesondere zwischen 18 und 35 liegt.

**2.** Verfahren nach Anspruch 1, dadurch gekennzeichnet, daß der Zeolith des Beta-Typs ein Atomverhältnis $Me^2/Me^1$ zwischen der Anzahl der Atome des Alkalimetalls $Me^2$ und der Anzahl der Atome aller dreiwertigen Elemente $Me^1$

des Zeoliths zwischen 0 und 0,2, insbesondere zwischen 0,005 und 0,1, hat.

**3.** Verfahren nach Anspruch 1, dadurch gekennzeichnet, daß der aromatische Ether der allgemeinen Formel (I) entspricht:

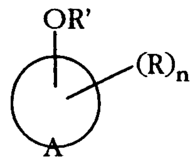

(I)

in der:

- A den Rest eines Rings bedeutet, der die Gesamtheit oder den Teil eines monocyclischen oder polycyclischen aromatischen Carboxylsystems bildet, das mindestens eine OR'-Gruppe hat: wobei der genannte cyclische Rest einen oder mehrere Substituenten tragen kann,
- R einen oder mehrere gleiche oder verschiedene Substituenten bedeutet,
- R' einen Kohlenwasserstoffrest mit 1 bis 24 Kohlenstoffatomen bedeutet, der ein linearer oder verzweigter gesättigter oder ungesättigter acyclischer aliphatischer Rest sein kann; einen monocyclischen oder polycyclischen cycloaliphatischen gesättigten, ungesättigten oder aromatischen Rest; einen linearen oder verzweigten gesättigten oder ungesättigten aliphatischen Rest, der einen cyclischen Substituenten trägt,
- R' und R einen Ring bilden können, der gegebenenfalls ein weiteres Heteroatom enthält,
- n eine Zahl kleiner oder gleich 4 ist.

**4.** Verfahren nach Anspruch 3, dadurch gekennzeichnet, daß der aromatische Ether der allgemeinen Formel (I) entspricht, in der R' bedeutet:

- einen linearen oder verzweigten, gesättigten oder ungesättigten, acyclischen aliphatischen Rest, vorzugsweise einen linearen oder verzweigten Alkylrest mit 1 bis 12 Kohlenstoffatomen, vorzugsweise mit 1 bis 6 Kohlenstoffatomen: wobei die Kohlenwasserstoffkette gegebenenfalls durch ein Heteroatom, eine funktionelle und/oder einen Substituenten tragende Gruppe unterbrochen sein kann,
- einen linearen oder verzweigten, gesättigten oder ungesättigten, aliphatischen acyclischen Rest, der Träger eines cyclischen und gegebenenfalls substituierten Substituenten ist: wobei der genannte acyclische Rest durch ein valentes Bindeglied, ein Heteroatom oder eine funktionelle Gruppe an den Ring gebunden sein kann,
- einen gesättigten oder 1 oder 2 ungesättigte Stellen im Ring enthaltenden carbocyclischen Rest mit üblicherweise 3 bis 8 Kohlenstoffatomen, vorzugsweise 6 Kohlenstoffatomen, im Ring; wobei der genannte Ring substituiert sein kann,
- einen vorzugsweise monocyclischen aromatischen carbocyclischen Rest mit üblicherweise mindestens 4 Kohlenstoffatomen, vorzugsweise 6 Kohlenstoffatomen im Ring; wobei der genannte Ring substituiert sein kann.

**5.** Verfahren nach Anspruch 3, dadurch gekennzeichnet, daß der aromatische Ether der allgemeinen Formel (I) entspricht, in der R' einen linearen oder verzweigten Alkylrest mit 1 bis 4 Kohlenstoffatomen, vorzugsweise einen Methyl- oder Phenylrest bedeutet oder in der die Reste R' und R einen Methylendioxy- oder Ethylendioxyrest bilden.

**6.** Verfahren nach einem der Ansprüche 3 bis 5, dadurch gekennzeichnet, daß der aromatische Ether der allgemein Formel (I) entspricht, in der der Rest A den Rest einer monocyclischen aromatischen carbocyclischen Verbindung mit wenigstens 4 Kohlenstoffatomen und vorzugsweise 6 Kohlenstoffatomen oder den Rest einer polycyclischen carbocyclischen Verbindung, vorzugsweise einen Naphthalinrest bedeutet: wobei der Rest A einen oder mehrere Substituenten an dem aromatischen Ring tragen kann.

**7.** Verfahren nach Anspruch 1, dadurch gekennzeichnet, daß der aromatische Ether der Formel (Ia) entspricht:

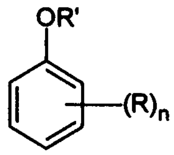

(Ia)

in der:

- n eine Zahl kleiner oder gleich 4, vorzugsweise gleich 0 oder 1, ist,
- der Rest R' einen linearen oder verzweigten Alkylrest mit 1 bis 6 Kohlenstoffatomen, vorzugsweise 1 bis 4 Kohlenstoffatomen, wie Methyl, Ethyl, Propyl, Isopropyl, Butyl, Isobutyl, sec.-Butyl, tert.-Butyl oder einen Phenylrest bedeutet,
- der oder die Reste R eine der folgenden Atome oder Gruppen bedeuten:
  - einen linearen oder verzweigten Alkylrest mit 1 bis 6 Kohlenstoffatomen, vorzugsweise 1 bis 4 Kohlenstoffatomen, wie Methyl, Ethyl, Propyl, Isopropyl, Butyl, Isobutyl, sec.-Butyl, tert.-Butyl,
  - einen linearen oder verzweigten Alkenylrest mit 2 bis 6 Kohlenstoffatomen, vorzugsweise 2 bis 4 Kohlenstoffatomen, wie Vinyl, Allyl,
  - einen Cyclohexyl- oder Benzylrest,
  - einen linearen oder verzweigten Alkoxyrest mit 1 bis 6 Kohlenstoffatomen, vorzugsweise 1 bis 4 Kohlenstoffatomen, wie die Methoxy-, Ethoxy-, Propoxy-, Isopropoxy-, Butoxyreste,
  - eine Acylgruppe mit 2 bis 6 Kohlenstoffatomen,
  - einen Rest der Formel:
    - $R_1$-OH
    - $R_1$-$COOR_2$
    - $R_1$-CHO
    - $R_1$-$NO_2$
    - $R_1$-CN
    - $R_1$-$N(R_2)_2$
    - $R_1$-CO-$N(R_2)_2$
    - $R_1$-X
    - $R_1$-$CF_3$

    wobei in den genannten Formeln $R_1$ ein valentes Bindeglied oder einen linearen oder verzweigten, gesättigten oder ungesättigten, zweiwertigen Kohlenwasserstoffrest mit 1 bis 6 Kohlenstoffatomen bedeutet, wie z.B. Methylen, Ethylen, Propylen, Isopropylen, Isopropyliden; $R_2$ ein Wasserstoffatom oder einen linearen oder verzweigten Alkylrest mit 1 bis 6 Kohlenstoffatomen bedeutet; X ein Halogenatom, vorzugsweise ein Chlor-, Brom- oder Fluoratom bedeutet;
- die Reste R' und R und die 2 aufeinanderfolgenden Atome des Benzolrings untereinander einen Ring mit 5 bis 7 Atomen bilden, die gegebenenfalls ein anderes Heteroatom enthalten.

**8.** Verfahren nach Anspruch 7, dadurch gekennzeichnet, daß der aromatische Ether der Formel (Ia) entspricht, in der n größer oder gleich 1 ist, die Reste R' und R und die 2 aufeinanderfolgenden Atome des Benzolrings untereinander durch einen Alkylen-, Alkenylen- oder Alkenylidenrest mit 2 bis 4 Kohlenstoffatomen verbunden sein können, um einen gesättigten, ungesättigten oder aromatischen heterocyclischen Ring mit 5 bis 7 Kohlenstoffatomen zu bilden, indem 1 oder mehrere Kolenstoffatome durch ein Heteroatom, vorzugsweise Sauerstoff, ersetzt sein können: wobei die Reste OR' und R vorzugsweise einen Methylendioxy- oder Ethylendioxyrest bilden.

**9.** Verfahren nach Anspruch 3 oder 7, dadurch gekennzeichnet, daß der aromatische Ether ein aromatischer Ether entsprechend der Formel (I) oder (Ia) ist, in denen:

- n gleich 0 oder 1 ist,
- R' einen linearen oder verzweigten Alkylrest mit 1 bis 6 Kohlenstoffatomen oder einen Phenylrest bedeutet,
- R einen linearen oder verzweigten Alkoxyrest mit 1 bis 4 Kohlenstoffatomen, vorzugsweise einen Methoxy-

oder Ethoxyrest, bedeutet,

- die Reste OR' und R einen Methylendioxy- oder Ethylendioxyrest bilden.

**10.** Verfahren nach Anspruch 3 oder 7, dadurch gekennzeichnet, daß der aromatische Ether ein nicht substituierter aromatischer Ether ist, der der Formel (I) oder (Ia) entspricht, in denen n gleich 0 ist; der Rest R' vorzugsweise einen linearen oder verzweigten Alkylrest mit 1 bis 6 Kohlenstoffatomen, vorzugsweise mit 1 bis 4 Kohlenstoffatomen, wie Methyl, Ethyl, Propyl, Isopropyl, Butyl, Isobutyl, sec.-Butyl, tert.-Butyl oder einen Phenylrest bedeutet, wobei der genannte Ether vorzugsweise Anisol ist.

**11.** Verfahren nach einem der Ansprüche 1 bis 10, dadurch gekennzeichnet, daß das Acylierungsmittel der Formel (II) entspricht:

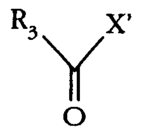

(II)

in der:

- $R_3$ bedeutet:
  - einen linearen oder verzweigten, gesättigten oder ungesättigten aliphatischen Rest mit 1 bis 24 Kohlenstoffatomen; einen monocyclischen oder polycyclischen, gesättigten oder ungesättigten cycloaliphatischen Rest mit 3 bis 12 Kohlenstoffatomen;
- X' bedeutet:
  - ein Halogenatom, vorzugsweise ein Chlor- oder Bromatom,
  - einen -O-CO-$R_4$-Rest mit $R_4$, gleich mit oder unterschiedlich zu $R_3$, mit derselben Bedeutung wie $R_3$: wobei $R_3$ und $R_4$ zusammen einen linearen oder verzweigten, gesättigten oder ungesättigten aliphatischen zweiwertigen Rest mit mindestens 2 Kohlenstoffatomen bilden können.

**12.** Verfahren nach Anspruch 11, dadurch gekennzeichnet, daß das Acylierungsmittel der Formel (II) entspricht, in der X' ein Chloratom und $R_3$ einen linearen oder verzweigten Alkylrest mit 1 bis 12 Kohlenstoffatomen, vorzugsweise 1 bis 4 Atomen, bedeutet: wobei die Kohlenwasserstoffkette gegebenenfalls durch ein Heteroatom oder durch eine funktionelle oder Substituenten, vorzugsweise Halogenatome, tragende Gruppe unterbrochen sein kann; X' einen -O-CO-$R_4$- Rest bedeutet, wobei $R_3$ und $R_4$ gleich sind und einen Alkylrest mit 1 bis 4 Kohlenstoffatomen bedeuten, die gegebenenfalls Träger von Halogenatomen sind.

**13.** Verfahren nach Anspruch 11 oder 12, dadurch gekennzeichnet, daß das Acylierungsmittel gewählt ist aus:

- Essigsäureanhydrid,
- Propionsäureanhydrid
- Isobuttersäureanhydrid
- Trifluoressigsäureanhydrid
- Monochloracetylanhydrid
- Dichloracetylanhydrid
- Acetylchlorid
- Monochloracetylchlorid
- Dichloracetylchlorid
- Propionsäurechlorid
- Isobuttersäurechlorid
- Pivaloylchlorid
- Crotonoylchlorid.

**14.** Verfahren nach einem der Ansprüche 1 bis 13, dadurch gekennzeichnet, daß der Katalysator ein Beta-Zeolith mit der chemischen Zusammensetzung im dehydratisierten Zustand entsprechend der folgenden empirischen Formel

ist:

$$(1\text{-}x)H_2O,\ x\ [(Me^2)_2O],\ [(Me^1)_2O_3],\ 2n\ SiO_2\ (F)$$

wobei in der genannten Formel:

- $Me^1$ alle Elemente mit einem Oxidationsgrad von +3 bedeutet, vorzugsweise Aluminium, Gallium, Eisen, Bor und ihre Gemische,
- $Me^2$ ein Metall aus der Gruppe 1a der Elemente und ihrer Gemische gewählt ist, vorzugsweise den Alkalimetallen wie Lithium, Natrium, Kalium, Rubidium und Cesium bedeutet,
- n, das zuvor definierte Atomverhältnis "Si/$Me^1$ global", zwischen 15 und 55, vorzugsweise zwischen 18 und 35, liegt,
- x, das Atomverhältnis $Me^2$/$Me^1$ zwischen der Anzahl der Atome des Alkalimetalls $Me^2$ und der Anzahl der Atome aller dreiwertigen Elemente $Me^1$ des Zeoliths, zwischen 0 und 0,2, vorzugsweise zwischen 0,005 und 0,1, liegt.

**15.** Verfahren nach Anspruch 14, dadurch gekennzeichnet, daß der Katalysator ein Zeolith entsprechend der chemischen Formel (F) ist, in der $Me^1$ Aluminium und $Me^2$ Natrium und/oder Kalium bedeutet.

**16.** Verfahren nach einem der Ansprüche 1 bis 15, dadurch gekennzeichnet, daß ein Beta-Zeolith einer solchen Entaluminierung unterzogen wird, daß das zuvor definierte Atomverhältnis "Si/$Me^1$ global" im genannten Bereich liegt.

**17.** Verfahren nach Anspruch 16, dadurch gekennzeichnet, daß die Entaluminierungen Calcinierungen in Gegenwart von Dampf, Calcinierungen in Gegenwart von Wasserdampf gefolgt von Angriffen mineralischer Säuren ($HNO_3$, HCl...), Entaluminierungen durch Reagenzien wie Siliciumtetrachlorid ($SiCl_4$), Ammoniumhexafluorsilikat ($(NH_4)_2SiF_6$) oder auch Ethylendiamintetraessigsäure (EDTA) sowie seiner Mono- oder Dinatriumform sind; wobei der direkte Säureangriff durch Lösungen mineralischer oder organischer Säuren oder durch alle Kombinationen der genannten Verfahren durchgeführt wird.

**18.** Verfahren nach einem der Ansprüche 1 bis 17, dadurch gekennzeichnet, daß der Zeolith allein oder als Gemisch mit einer mineralischen Matrix eingesetzt wird, die vorzugsweise aus den Metalloxiden wie den Aluminium-, Silicium- und/oder Zirconiumoxiden gewählt ist oder auch aus den Tonen und noch spezieller Kaolin, Talk oder Montmorillonit.

**19.** Verfahren nach einem der Ansprüche 1 bis 18, dadurch gekennzeichnet, daß man ein organisches Lösungsmittel einsetzt, gewählt aus:

- den alipahtischen, cycloaliphatischen oder aromatischen Kohlenwasserstoffen,
- den halogenierten aliphatischen oder aromatischen Kohlenwasserstoffen,
- den aliphatischen, cycloaliphatischen oder aromatischen Etheroxiden,
- den linearen oder cyclischen Carboxamiden,
- Dimethylsulfoxid (DMSO),
- den cyclischen oder nicht cyclischen Sulfonen,
- Hexamethylphosphotriamid (HMPT),
- den cyclischen oder nicht cyclischen tetrasubstituierten Harnstoffen.

**20.** Verfahren nach einem der Ansprüche 1 bis 19, dadurch gekennzeichnet, daß das Verhältnis zwischen der Molzahl des aromatischen Ethers und der Molzahl des Acylierungsmittels zwischen 0,1 und 20, vorzugsweise zwischen 0,5 und 10, liegt.

**21.** Verfahren nach einem der Ansprüche 1 bis 20, dadurch gekennzeichnet, daß die Menge des Katalysators bezogen auf das Gewicht im Verhältnis zum verwendeten aromatischen Ether 0,01 bis 50 %, vorzugsweise 1,0 bis 20 %, beträgt.

**22.** Verfahren nach einem der Ansprüche 1 bis 21, dadurch gekennzeichnet, daß die Temperatur, bei der die Acylierungsreaktion durchgeführt wird, zwischen 20 °C und 300 °C, vorzugsweise zwischen 40 °C und 200 °C, liegt.

## Claims

1. A process for the acylation of an aromatic ether which consists of reacting said ether with an acylation agent in the presence of a zeolitic catalyst, characterized in that the acylation reaction is carried out by reacting the aromatic ether with an acylation agent selected from aliphatic or cycloaliphatic carboxylic acid halides and/or anhydrides in the presence of an effective quantity of a catalyst comprising a beta zeolite with an atomic ratio denoted "global $Si/Me^1$" between the number of atoms of the element silicon and the number of atoms of every trivalent element $Me^1$ contained in the zeolite of no less than 15, preferably in the range 15 to 55, and more preferably in the range 18 to 35.

2. A process according to claim 1, characterized in that the beta zeolite has an atomic ratio $Me^2/Me^1$ between the number of alkali metal atoms $Me^2$ and the number of atoms of any trivalent element $Me^1$ of the zeolite is in the range 0 to 0.2, preferably in the range 0.005 to 0.1.

3. A process according to claim 1, characterized in that the aromatic ether has general formula (I):

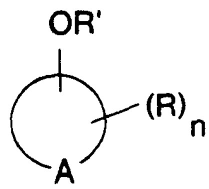

(I)

where:

- A represents the residue of a cycle forming all or a portion of a monocyclic or polycyclic aromatic carbocyclic system containing at least one OR' group: the cyclic residue may carry one or more substitutents;
- R represents one or more substitutents which may be identical or different;
- R' represents a hydrocarbon radical containing 1 to 24 carbon atoms, which may be a saturated or unsaturated, linear or branched acyclic aliphatic radical; a monocyclic or polycyclic, saturated, unsaturated or aromatic cycloaliphatic radical; or a saturated or unsaturated, linear or branched aliphatic radical carrying a cyclic substitutent;
- R' and R may form a cycle which may contain a further heteroatom;
- n is a number less than or equal to 4.

4. A process according to claim 3, characterized in that the aromatic ether has general formula (I) where R' represents:

- a saturated or unsaturated, linear or branched acyclic aliphatic radical, preferably a linear or branched alkyl radical containing 1 to 12 carbon atoms, preferably 1 to 6 carbon atoms; the hydrocarbon chain may be interrupted by a heteroatom, a functional group and/or may carry a substitutent;
- a saturated or unsaturated, linear or branched acyclic aliphatic radical carrying a cyclic substitutent which may be substituted: said acyclic radical may be bonded to the cycle by a valence bond, a heteroatom or a functional group;
- a carbocyclic radical which may be saturated or contain 1 or 2 unsaturations in the cycle, generally containing 3 to 8 carbon atoms, preferably 6 carbon atoms in the cycle; said cycle may be substituted;
- an aromatic carbocyclic radical, preferably a monocyclic radical generally containing at least 4 carbon atoms, preferably 6 carbon atoms in the cycle; said cycle may be substituted.

5. A process according to claim 3, characterized in that the aromatic ether has general formula (I) in which R' represents a linear or branched alkyl radical containing 1 to 4 carbon atoms, preferably a methyl, ethyl or phenyl radical, or in which radicals R and R' form a dioxymethylene or dioxyethylene radical.

6. A process according to any one of claims 3 to 5, characterized in that the aromatic ether has general formula (I) where residue A represents the residue of a monocyclic aromatic carbocyclic compound containing at least 4 carbon atoms and preferably 6 carbon atoms or the residue of a polycyclic carbocyclic compound, preferably a naphthalenic residue: residue A may carry one or more substitutents on the aromatic nucleus.

**7.** A process according to claim 1, characterized in that the aromatic ether has formula (Ia):

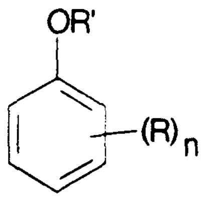

(Ia)

where:

- n is a number less than or equal to 4, preferably 0 or 1;
- radical R' represents a linear or branched alkyl radical containing 1 to 6 carbon atoms. preferably 1 to 4 carbon atoms, such as methyl, ethyl, propyl, isopropyl, butyl, isobutyl, sec-butyl, tert-butyl or a phenyl radical;
- radical(s) R represent one of the following atoms or groups:
  - a linear or branched alkyl radical containing 1 to 6 carbon atoms, preferably 1 to 4 carbon atoms, such as methyl, ethyl, propyl, isopropyl, butyl, isobutyl, sec-butyl or tert-butyl;
  - a linear or branched alkenyl radical containing 2 to 6 carbon atoms, preferably 2 to 4 carbon atoms, such as vinyl, allyl;
  - a cyclohexyl or benzyl radical;
  - a linear or branched alkoxy radical containing 1 to 6 carbon atoms, preferably 1 to 4 carbon atoms, such as a methoxy, ethoxy, propoxy, isopropoxy or butoxy radical;
  - an acyl group containing 2 to 6 carbon atoms;
  - a radical with formula:
    - $-R_1-OH$
    - $-R_1-COOR_2$
    - $-R_1-CHO$
    - $-R_1-NO_2$
    - $-R_1-CN$
    - $-R_1-N-(R_2)_2$
    - $-R_1-CO-N-(R_2)_2$
    - $-R_1-X$
    - $-R_1-CF_3$

    where $R_1$ represents a valence bond or a saturated or unsaturated, linear or branched divalent hydrocarbon radical containing 1 to 6 carbon atoms, such as methylene, ethylene, propylene, isopropylene, or isopropylidene;
    $R_2$ represents a hydrogen atom or a linear or branched alkyl radical containing 1 to 6 carbon atoms; and X represents a halogen atom, preferably a chlorine, bromine or fluorine atom;
- radicals R' and R and the 2 successive atoms of the benzene ring may form between them a cycle containing 5 to 7 carbon atoms, which may contain a further heteroatom.

**8.** A process according to claim 7, characterized in that the aromatic ether has formula (Ia) where n is greater than or equal to 1, radicals R and R' and the 2 successive atoms of the benzene ring can be bonded together by an alkylene, alkenylene or alkenylidene radical containing 2 to 4 carbon atoms to form a saturated, unsaturated or aromatic heterocycle containing 5 to 7 carbon atoms in which one or more carbon atoms can be replaced by a heteroatom, preferably oxygen: the radicals OR' and R preferably forming a dioxymethylene or dioxyethylene radical.

**9.** A process according to claims 3 to 7, characterized in that the aromatic ether is an aromatic ether with formula (I) or (Ia) where:

- n is 0 or 1;
- R' represents a linear or branched alkyl radical containing 1 to 6 carbon atoms or a phenyl radical;

- R represents a linear or branched alkoxy radical containing 1 to 4 carbon atoms, preferably a methoxy or ethoxy radical;
- radicals OR' and R form a dioxymethylene or dioxyethylene radical.

**10.** A process according to claims 3 to 7, characterized in that the aromatic ether is an unsubstituted aromatic ether with formula (I) or (Ia) where n equals 0; radical R' preferably represents a linear or branched alkyl radical containing 1 to 6 carbon atoms, preferably 1 to 4 carbon atoms, such as methyl, ethyl, propyl, isopropyl, butyl, isobutyl, sec-butyl, tert-butyl or a phenyl radical, said ether preferably being anisole.

**11.** A process according to any one of claims 1 to 10, characterized in that the acylation agent has formula (II):

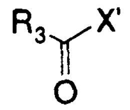

(II)

where:

- $R_3$ represents:
  - a saturated or unsaturated, linear or branched aliphatic radical containing 1 to 24 carbon atoms; or a saturated or unsaturated, monocyclic or polycyclic cycloaliphatic radical containing 3 to 12 carbon atoms;
- X' represents:
  - a halogen atom, preferably a chlorine or bromine atom;
  - a -O-CO-$R_4$ radical where $R_4$, which may be identical or different to $R_3$, has the same meaning as $R_3$; $R_3$ and $R_4$ may together form a saturated or unsaturated, linear or branched aliphatic radical containing at least 2 carbon atoms.

**12.** A process according to claim 11, characterized in that the acylation agent has formula (II) where X' represents a chlorine atom and $R_3$ represents a linear or branched alkyl radical containing 1 to 12 carbon atoms, preferably 1 to 4 atoms: the hydrocarbon chain may be interrupted by a heteroatom or by a functional group or may carry substitutents, preferably halogen atoms; or X' represents a -O-CO-$R_4$ radical, where $R_3$ and $R_4$ are identical and represent an alkyl radical containing 1 to 4 carbon atoms which may carry halogen atoms.

**13.** A process according to claim 11 or claim 12, characterized in that the acylation agent is selected from:

- acetic anhydride;
- propanoic anhydride;
- isobutyric anhydride;
- trifluoroacetic anhydride;
- monochloroacetyl anhydride;
- dichloroacetyl anhydride;
- acetyl chloride;
- monochloroacetyl chloride;
- dichloroacetyl chloride;
- propanoyl chloride;
- isobutanoyl chloride;
- pivaloyl chloride;
- crotonyl chloride.

**14.** A process according to any one of claims 1 to 13, characterized in that the catalyst is a β zeolite having, in its dehydrated state, a chemical composition corresponding to the following empirical formula:

$$(1\text{-}x)H_2O,\ x[(Me^2)_2O],[(Me^1)_2O_3],\ 2nSiO_2\ (F)$$

where:

- $Me^1$ represents any element with a degree of oxidation of +3, preferably aluminium, gallium, iron, boron and mixtures thereof;
- $Me^2$ represents a metal selected from the group of elements from column 1a and mixtures thereof, preferably alkali metals such as lithium, sodium, potassium, rubidium and caesium;
- n, equal to the "global Si/$Me^1$" atomic ratio defined above is in the range 15 to 55, preferably in the range 18 to 35;
- x, equal to the $Me^2/Me^1$ atomic ratio defined above, between the number of atoms of alkali metal $Me^2$ and the numbr of atoms of every trivalent element $Me^1$ in the zeolite, is in the range 0 to 0.2, preferably in the range 0.005 to 0.1.

**15.** A process according to claim 14, characterized in that the catalyst is a zeolite with chemical formula (F) where $Me^1$ represents aluminium and $Me^2$ represents sodium and/or potassium.

**16.** A process according to any one of claims 1 to 15, characterized in that the β zeolite undergoes dealuminising treatment so that the "global Si/$Me^1$" atomic ratio defined above is in the range defined above.

**17.** A process according to claim 16, characterized in that the dealuminising treatment is calcining in the presence of vapour, calcining in the presence of steam followed by attack with mineral acids ($HNO_3$, HCl...), dealuminising using reactants such as silicon tetrachloride ($SiCl_4$), ammonium hexafluorosilicate ($(NH_4)_2SiF_6$) or ethylenediaminetetracetic acid (EDTA) or its mono or disodium form; or direct acid attack using solutions of mineral acids or organic acids, or any combination of said methods.

**18.** A process according to any one of claims 1 to 17, characterized in that the zeolite is used alone or mixed with a mineral matrix which is preferably selected from metal oxides such as aluminium, silicon and/or zirconium oxides, or from clays, in particular kaolin, talc or montmorillonite.

**19.** A process according to any one of claims 1 to 18, characterized in that an organic solvent is used which is selected from:

- aliphatic, cycloaliphatic or aromatic hydrocarbons;
- aliphatic or aromatic halogenated hydrocarbons;
- aliphatic, cycloaliphatic or aromatic ether-oxides;
- linear or cyclic carboxamides;
- dimethylsulphoxide (DMSO);
- cyclic or non cyclic sulphones;
- hexamethylphosphotriamide (HMPT);
- cyclic or non cyclic tetrasubstituted ureas.

**20.** A process according to any one of claims 1 to 19, characterized in that the ratio between the number of moles of aromatic ether and the number of moles of acylation agent is between 0.1 and 20, preferably between 0.5 and 10.

**21.** A process according to any one of claims 1 to 20, characterized in that the quantity of catalyst represents 0.01% to 50% by weight, preferably 1.0% to 20% by weight of the aromatic ether employed.

**22.** A process according to any one of claims 1 to 21, characterized in that the temperature at which the acylation reaction is carried out is between 20°C and 300°C, preferably between 40°C and 200°C.